# EUROPÄISCHE PATENTSCHRIFT

(11) **EP 1 152 182 B1**
(45) Veröffentlichungstag und Bekanntmachung des Hinweises auf die Patenterteilung: **11.01.2006**
(21) Anmeldenummer: 00108786.5
(22) Anmeldetag: 25.04.2000
(51) Int. Cl.: F16M 11/12, G02B 7/00

(54) **Stativ mit einem Operationsmikroskop**
Stand with a surgical microscope
Support avec un microscope chirurgical

(43) Veröffentlichungstag der Anmeldung: 07.11.2001
(73) Patentinhaber: Möller-Wedel GmbH, 22880 Wedel (DE)
(72) Erfinder: Twisselmann, Lorenz, Dr., 25497 Prisdorf (DE)
(74) Vertreter: Glawe, Delfs, Moll

(56) Entgegenhaltungen:
- EP-A- 0 552 524
- EP-A- 0 849 053
- DE-A- 4 320 443
- DE-A- 4 334 069
- DE-A- 19 732 212
- US-A- 5 213 293
- US-A- 5 273 039
- US-A- 5 332 181

## Beschreibung

Die Erfindung betrifft ein Operationsmikroskop mit einem Stativ, wobei das Mikroskop insgesamt bis zu sechs Freiheitsgrade aufweist und über Elemente des Stativs an der Mikroskopaufhängung befestigt ist, wobei das Stativ zwei vertikale Schwenkachsen, die keinem Einfluß durch Schwerkraft unterworfen sind, und für die Höhenverstellung ein Parallelogrammgestänge mit Gewichtsausgleich aufweist, die mit leichtgängiger Beweglichkeit und mit Bremsen für die Arretierung ausgebildet sind.

Bei einem solchen Stativ (US 5,213,293 A) kann das Mikroskop mit Hilfe des Stativs in die für die Operation gewünschte Stellung gebracht werden. Es weist einen Gewichtsausgleich auf, so daß es auch bei schwach gebremsten Gelenken in dieser Stellung verbleibt. Es ist aber sehr aufwendig, diesen Gewichtsausgleich zu bewirken. Eine anschließende Verstellung des Mikroskops, um das Bildfeld zu ändern, muß per Hand erfolgen.

Bei einem anderen bekannten Mikroskop (US 5,332,181) findet die Verstellung um alle Achsen motorisch statt. Dazu ist nur ein grober Gewichtsausgleich erforderlich, was selbstverständlich leichter zu bewirken ist, als der feine Ausgleich der erstgenannten Anordnung. Das Problem bei der Einstellung des Mikroskops auf eine neue Aufgabe bzw. neue Bedingungen erfolgt durch die Motoren nur sehr langsam, da schnelle automatische Bewegungen um die einzelnen Freiheitsgrade zu große Motoren erfordern würden und insbesondere auch gefährlich sind. Der Operateur muß daher sehr lange warten, bis die von ihm gewünschte Einstellung erreicht worden ist. Dadurch wird der Vorteil, daß nur eine grobe Ausbalancierung erforderlich ist, zunichte gemacht.

Zur manuellen Handhabung eines OP-Mikroskops an einem Stativ ist eine Ausbalancierung immer erforderlich. Es ist nicht nur ein Gewichtsausgleich durch Gegengewichte, Federn und Gasfedern notwendig, da die Arme des Stativs ein gewisses Gewicht haben, das natürlich immer den gleichen Wert hat. Bei Verstellungen an Zubehörteilen des Mikroskops, die bei der Ausführung von Operationen notwendig werden, verschiebt sich aber der Schwerpunkt am Mikroskop. Es muß dann eine erneute Gewichtskompensation um die Drehachsen vorgenommen werden. Der mechanische Aufwand bei mehreren Drehachsen ist unter Berücksichtigung der notwendigen Steifigkeit der Mikroskopaufhängung erheblich und wird durch motorische Antriebe noch erhöht, wenn man dem Anwender die Gewichtskompensation erleichtern will. Es sind sogar Ausführungen bekannt (DE 4320443 A, DE 43 34 069 A), bei denen die Gewichtskompensation mit Sensoren, Reglern und Stellgliedern auf Abruf automatisch durchgeführt werden.

Es ist ein Stativ 8 mit einem Operationsmikroskop bekannt (EP 849 053 A), wobei das Mikroskop 2 insgesamt bis zu sechs Freiheitsgrade aufweist und über Elemente A1-A7 des Stativs 8 an der Mikroskopaufhängung befestigt ist, die zwei vertikale Schwenkachsen C1, C4, die keinem Einfluss durch Schwerkraft unterworfen sind, und für die Höhenverstellung ein Parallelogrammgestänge A3 mit Gewichtsausgleich aufweist, wobei das Mikroskop 2 über eine dritte vertikale Achse C5, die keinen Einfluss durch Schwerkraft unterworfen ist, und eine erste weitere dazu senkrechte Achse C6 am Stativ 8 gelagert ist, wobei die weitere Achse C6 im wesentlichen senkrecht zur optischen Achse des Mikroskopobjektivs und motorisch verstellbar ausgebildet ist und eine Schwenkung der Blickrichtung vor/zurück oder nach oben/unten ermöglicht. Bei diesem Mikroskop werden die Achsen motorisch durch Betätigung eines Bedienungshandgriffs bewegt.

Die Aufgabe der Erfindung besteht in der Schaffung eines Mikroskops mit einem Stativ, bei dem die Einstellung des Ortes des Mikroskops schnell von Hand vorgenommen werden kann, anschließend aber eine Feineinstellung des beobachtbaren Bildfeldes vorgenommen werden kann, ohne daß diese Handhabung durch Schwerkräfte oder dadurch verursachte Momente gestört wird.

Die erfindungsgemäße Lösung ist in Anspruch 1 angegeben.

Die erfindungsgemäße Lösung besteht darin, daß das Mikroskop über eine dritte vertikale Achse, die keinem Einfluß durch Schwerkraft unterworfen ist, eine erste dazu senkrechte Achse und eine zweite weitere zur ersten weiteren zur ersten weiteren senkrechte Achse am Stativ gelagert ist, wobei die weiteren Achsen im wesentlichen senkrecht zur optischen Achse des Mikroskopobjektivs und motorisch verstellbar ausgebildet sind und die dem Mikroskop nähere weitere Achse eine seitliche Schwenkung der Blickrichtung (X-Richtung) ermöglicht und die zwischen der dritten vertikalen Achse und der dem Mikroskop näheren weiteren Achse angeordnete weitere Achse eine Schwenkung der Blickrichtung vor/zurück oder nach oben/ unten (Y-Richtung) ermöglicht.

Es ist zwar bekannt, die Beweglichkeit von Operationsmikroskopen an Trägereinheiten mit Tasten an Handgriffen freizuschalten, die am Mikroskop angeordnet sind. Bei Betätigung der Tasten werden elektromotorische Bremsen der Stativgelenke gelöst. Die Erfindung erschöpft sich aber nicht darin, daß die Bewegung um diese Achsen schnell von Hand vorgenommen werden kann und dann die Achsen in dieser Stellung blockiert werden können. Die Erfindung zeichnet sich vielmehr durch eine Kombination dieser Verstellmöglichkeit von Hand mit einer motorischen Einstellung aus.

Die größeren Verstellwege des Stativs werden dem Anwender/Operateur in an sich bekannter Weise gewichtsausgeglichen im sogenannten "free floating modus" freigegeben. Er führt das Gerät an einem Handgriff bspw. mit einer integrierten Taste, mit der die Bewegung freigegeben werden kann. Dabei ist ein feiner Gewichtsausgleich am Parallelogrammarm vorgenommen worden. Dieser Gesamtgewichtsausgleich muß möglichst genau sein, so daß für die Einstellungen nur sehr geringe Kräfte erforderlich sind. Ein Ungleichgewicht an den beiden direkt am Mikroskop vorhandenen Einstellachsen stört dann nicht, wenn bei diesen Achsen eine motorische Verstellung vorgesehen ist und die Motoren kräftig genug sind, die entsprechenden Kräfte des Ungleichgewichts aufzunehmen.

Durch die Drehungen um die dritte vertikale Achse sowie die beiden weiteren Achsen kann der Operateur das Blickfeld an die Erfordernisse anpassen. Durch die besondere Reihenfolge der beiden weiteren Achsen ist dabei eine sehr zweckmäßige Verstellbarkeit für unterschiedliche Operationsbedingungen gegeben. Die optische Achse des Operationsmikroskops wird nämlich für unterschiedliche Operationen (z. B. Gehirnoperationen, Rückenmarkoperationen, Augenoperationen) völlig unterschiedlich zur Vertikalen ausgerichtet. Wählt man die Reihenfolge der weiteren Achse anders, so ist diese günstige Veränderung des Blickfeldes nicht mehr möglich.

Zweckmäßigerweise sind die motorischen Antriebe als Servoantriebe ausgebildet. Die Vorgabe für diese Servofunktionen erfolgt in bevorzugter Weise von elektrischen Kraft- oder Momentensensoren zwischen dem Mikroskop und dem Bedienungselement für die entsprechenden Richtungen. Die Sensoren sind so ausgeführt, daß sie ein vom Benutzer mit dem am Bedienungselement um eine Achse ausgeübtes Moment steigendes und bei Richtungswechsel im Vorzeichen wechselndes Signal abgeben. Zweckmäßigerweise sind die Bedienungselemente verstellbar, so daß der Benutzer sie in die für ihn bequemste Lage verstellen kann, ohne daß dabei die Lage der Sensoren zum Mikroskop und damit der Bezug zwischen den Signalen und Wirkrichtung verändert wird.

Bei Längs- und Querkräften und bei Momenten senkrecht zur Sensorachse wird kein Signal abgegeben. Bei einer besonders zwechmäßigen Ausführungsform sind die Sensoren paarweise so angeordnet, daß, wenn durch eine von einer Bedienungsperson auf das Bedienungselement ausgeübte Kraft der eine Sensor entlastet wird, der andere belastet wird. Beide Sensoren sind bezüglich des zu detektierenden Moments bzw. der zu detektierenden Kraft beiderseits einer neutralen Linie angeordnet. Beide Sensorelemente stehen unter einem Druck und sind in einer Brückenschaltung gegeneinander geschaltet, so daß sich die Signale durch den Druck gegenseitig aufheben. Wird ein Moment oder eine Kraft in das Bedienungselement eingeleitet, das bzw. die eine Drehung um diese Linie zu bewirken versucht, entsteht auf der einen Seite der neutralen Linie steigender Druck, auf der anderen Seite fallender Druck. Entsprechend wird die Brücke verstimmt und liefert ein dem Moment entsprechendes Ausgangssignal. Als Sensoren können Piezoelemente, induktive Sensoren, kapazitive Aufnehmer, resistive und optische Kraft/Wegaufnehmer verwendet werden.

Die Signale werden steuerungstechnisch weiterverarbeitet, um über Motortreiber und Servomotoren die Verstellungen vorzunehmen. Bei einer ausreichenden Dynamik der beiden Servosteuerungen zusammen mit dem "free floating" der anderen vier Freiheitsgrade werden die Einstellzeiten verkleinert. Während der Anwender das Mikroskop in den vier zur Bewegung freigegebenen Freiheitsgraden verstellt, wirken gleichzeitig die zur Richtungseinstellung der Mikroskopachse eingeleiteten Momente am Bedienungselement und bewirken mit den Servosteuerungen die entsprechende Verstellung. Durch die synchrone freie Verstellung des Mikroskops mit größeren Wegen und den Servonachlauf der kleinen Drehbewegungen entfällt der von reinen Servosystemen bekannte Effekt, daß der Anwender lange warten muß, bis die Verstellung beendet ist.

Zweckmäßigerweise ist die Anordnung so getroffen, daß die möglichen Bewegungen um die einzelnen Achsen bzw. Freiheitsgrade so begrenzt sind, daß das Mikroskop nicht gegen Teile des Stativs anstoßen kann.

Die Erfindung wird im folgenden anhand vorteilhafter Ausführungsformen unter Bezugnahme auf die beigefügten Zeichnungen beschrieben. Es zeigen:
- Fig. 1: den prinzipiellen Aufbau des erfindungsgemäßen Sta-tivs in einer Seitenansicht;
- Fig. 2: in Seitenansicht das Mikroskop;
- Fig. 3: in Frontansicht das Mikroskop;
- Fig. 4: in ähnlicher Darstellung wie in Fig. 2 das Mikroskop, nachdem es um 90° gedreht ist;
- Fig. 5: in ähnlicher Darstellung wie in Fig. 3 das Mikroskop, nachdem es um 90° gedreht ist; und
- Fig. 6: den prinzipiellen Aufbau der Sensoren und elektrischen Auswertungsschaltungen.

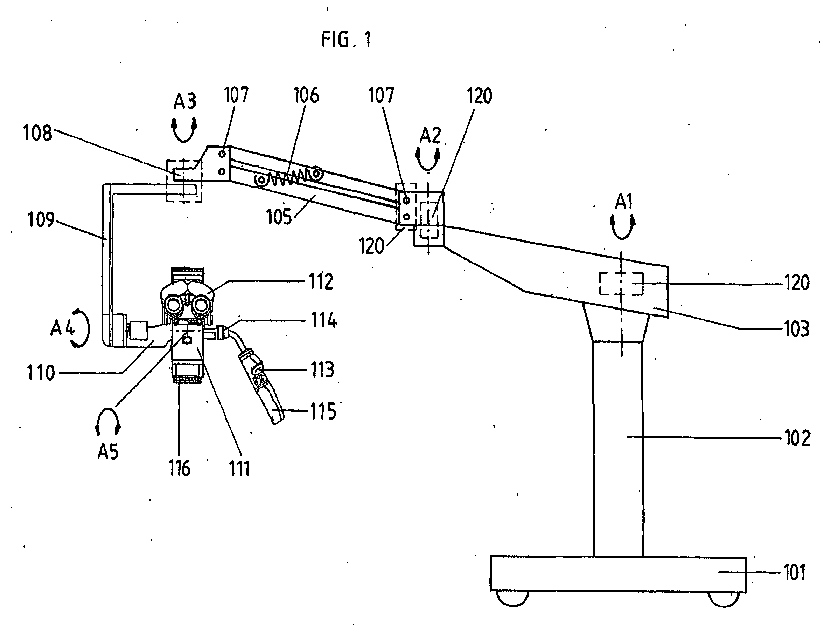

In Fig. 1 ist die Anordnung eines Operationsmikroskops an einem Fußbodenstativ dargestellt. Das Stativ steht auf einem Basisteil oder Fuß 101, der im allgemeinen mit Rollen zum Verfahren ausgestattet ist. Selbstverständlich kann der Basisteil 101 auch für Decken- oder Wandbefestigung ausgebildet sein. Auf diesem Basisteil 101 ist eine Säule 102 angebracht, um deren Achse A1 sich der aufgesetzte feste Arm 103 drehen läßt. Mit einem um die Achse A2 drehbaren Gelenk ist daran ein Parallelogrammarm 105 mit Achser 107 befestigt. Die Höhenverstellung des Mikroskopanschlusses 108 am Parallelogramm 105 ist mit einer Gasfeder oder einem Federpaket 106 gewichtsausgeglichen.

Das Mikroskop besteht aus einem Mikroskopkörper 111, dem Einblick 112 und dem Objektiv 116 und ist mit den Armen 110 und 109 drehbar um die Achse A3 am Mikroskopanschluß des Stativs befestigt. Es läßt sich mit den Drehungen um die Achsen A1, A2 und A3 sowie durch die gewichtsausgeglichene Höhenverstellung mit dem Parallelogrammarm 105 im mechanisch vorgegebenen Raum bis auf Reibungswiderstände frei von Schwerkrafteinfluß bewegen.

Wie dies in den Fig. 1-3 dargestellt ist, ist das dort dargestellte Mikroskop 111 mit dem Servoantrieb 204 um die Achse A5 drehbar. Dies entspricht einer Drehung im Sehfeld in seitlicher Richtung (X-Richtung). Zusammen mit dem Arm 110 kann das Mikroskop 111 vom Servoantrieb 206 um die Achse A4 gedreht werden. Dies entspricht einer Bewegung im Sehfeld nach oben/unten (Y-Richtung).

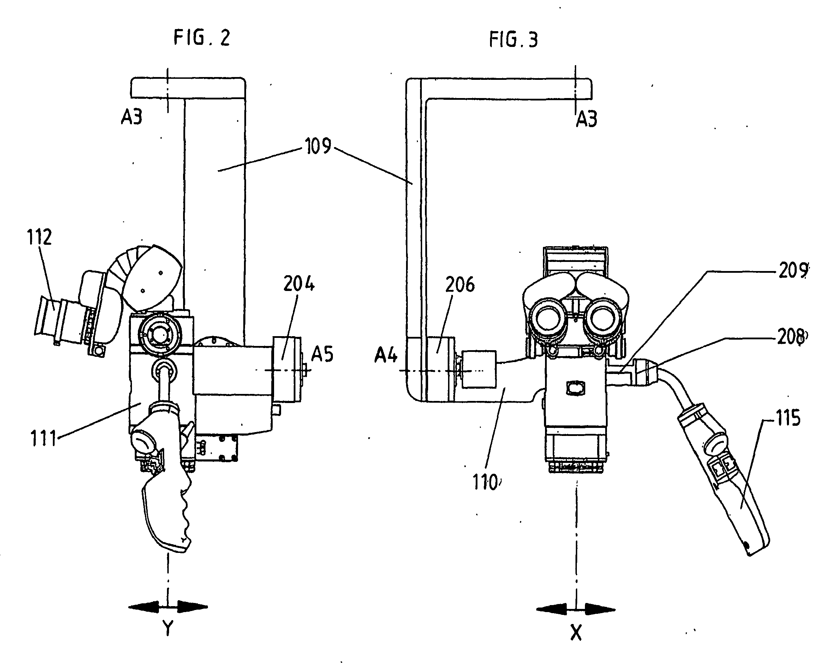

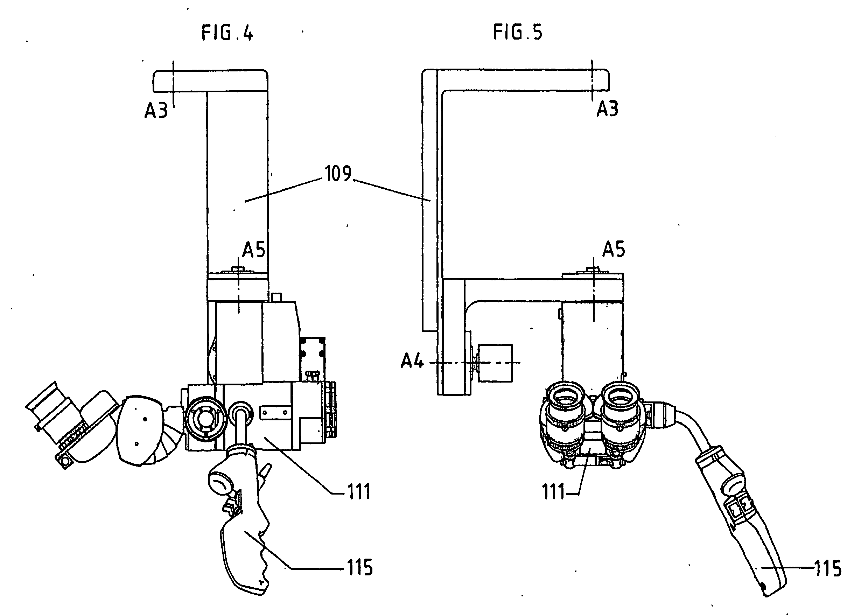

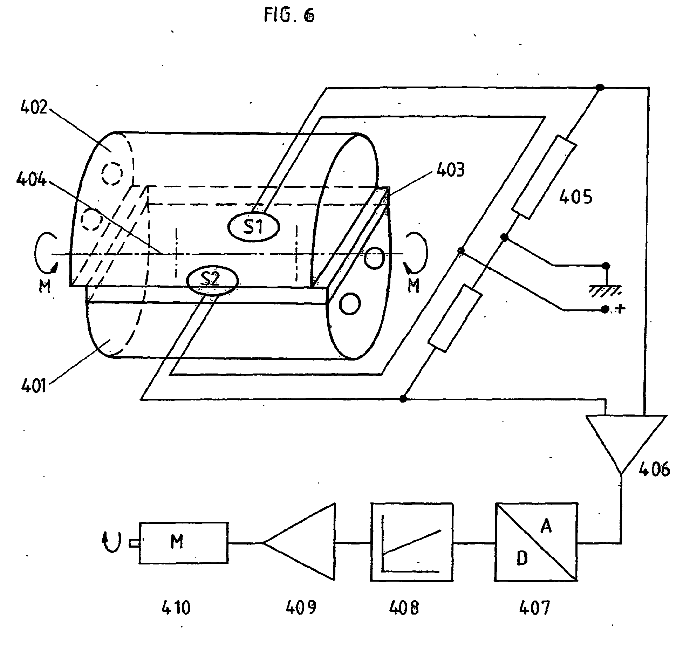

In den Fig. 4 und 5 ist die Anordnung in ähnlicher Darstellung wie in den Fig. 2 und 3 gezeigt, wobei das Mikroskop 111 allerdings um die Achse A4 um 90° gedreht ist, so daß die Objektivachse waagerecht ist.

In Fig. 6 ist schematisch und beispielhaft ein Kraft- und Momentsensor für die Y-Verstellung dargestellt. Zwischen den Körpern 401 und 402 ist eine elastische Platte 403 angespannt. Darin sind zwei Drucksensoren S1 und S2 angeordnet. Wenn zwischen den beiden Körpern 401 und 402 ein Moment M zur Wirkung kommt, so ergeben sich in der elastischen Platte 403 auf den beiden Seiten einer neutralen Phase 404 verstärkte und abgeschwächte Druckkräfte, die in den Sensoren S1 und S2 Widerstandsänderungen hervorrufen. Die Weiterverarbeitung der Signale erfolgt mit der Brückenschaltung 405, dem Verstärker 406, dem Analog/Digitalwandler 407, regelungstechnischer Bearbeitung 408 und einem Treiber 409 für den Schrittmotor 410 zur Servoverstellung der Achse A4.

Wie dies erwähnt wurde, ist die Bewegung um die Achsen A1, A2 und A3 sowie diejenige des Parallelogrammgestänges 105 leichtgängig, so daß einfach eine Verstellung vorgenommen werden kann. Arretierung findet dann durch die Bremsen 120 statt, die in Fig. 1 gestrichelt angedeutet sind. Diese Bremsen können für die Grobverstellung gelöst werden, indem eine entsprechende Taste 113 am Bedienungselement 115 betätigt wird. Der Antrieb der Motoren kann direkt auf die entsprechenden Achsen wirken. Es ist jedoch auch möglich, ein Getriebe zwischenzuschalten.

## Patentansprüche

1. Stativ mit einem Operationsmikroskop, wobei das Mikroskop (111) insgesamt bis zu sechs Freiheitsgrade aufweist und über Elemente des Stativs (102, 103, 106, 108) an einer Mikroskopaufhängung (109, 110) befestigt ist, die zwei vertikale Schwenkachsen (A1, A2), die keinem Einfluß durch Schwerkraft unterworfen sind, und für die Höhenverstellung ein Parallelogrammgestänge (105) mit Gewichtsausgleich aufweist, die mit leichtgängiger Beweglichkeit und, mit Bremsen für die Arretierung ausgebildet sind, wobei das Mikroskop über eine dritte vertikale Achse (A3), die keinem Einfluß durch Schwerkraft unterworfen ist, eine erste weitere dazu senkrechte Achse (A4), und eine zweite weitere zur ersten weiteren senkrechte Achse (A5) am Stativ gelagert ist, wobei die weiteren Achsen (A4, A5) im wesentlichen senkrecht zur optischen Achse des Mikroskopobjektivs (116) und motorisch verstellbar ausgebildet sind und die dem Mikroskop (111) nähere weitere Achse (A5) eine seitliche Schwenkung der Blickrichtung (X-Richtung) ermöglicht und die zwischen der dritten vertikalen Achse (A3) und der dem Mikroskop (111) näheren weitere Achse (A5) angeordnete weitere Achse (A4) eine Schwenkung der Blickrichtung vor/zurück oder nach oben/unten (Y-Richtung) ermöglicht,

2. Stativ nach Anspruch 1, **dadurch gekennzeichnet, daß** die motorischen Antriebe (410) als Servoantriebe ausgebildet sind.

3. Stativ nach Anspruch 2, **dadurch gekennzeichnet, daß** zur Steuerung der Servoantriebe zwischen Mikroskop (111) und mindestens einem Bedienungselement (115) Kraft- oder Drehmomentsensoren (S1, S2) angeordnet sind, die entsprechend der Größe und Richtung der auf die Bedienungselemente ausgeübten Kraft bzw. des ausgeübten Drehmoments Signale für die entsprechende Steuerung der Motoren (410) erzeugen.

4. Stativ nach Anspruch 3, **dadurch gekennzeichnet, daß** die Bedienungselemente (115) verstellbar sind, ohne daß die Lage der Sensoren (S1, S2) zum Mikroskop (111) verändert wird.

5. Stativ nach Anspruch 3 oder 4, **dadurch gekennzeichnet, daß** die Sensoren (S1, S2) paarweise so angeordnet sind, daß, wenn durch eine von einer Bedienungsperson auf das Bedienungselement (115) ausgeübte Kraft ein Sensor (S1, S2) entlastet wird, der andere (S2, S1) belastet wird.

6. Stativ nach Anspruch 5, **dadurch gekennzeichnet, daß** die Sensoren (S1, S2) in einer Brückenschaltung (405) angeordnet sind, die eine Richtung und Geschwindigkeit der motorischen Verstellung bestimmende Spannung abgibt.

7. Stativ nach Anspruch 6, **dadurch gekennzeichnet, daß** es Schaltungen (406-409) zum Umwandeln der Spannung in eine Versorungsspannung für den Servoantrieb (204, 206) aufweist.

8. Stativ nach Anspruch 6, **dadurch gekennzeichnet, daß** es Schrittmotoren (410) für den Servoantrieb (204, 206), Analog/Digitalwandler (407) für die Spannung und Signalaufbereitungsschaltungen (408, 409) für die Ausgangssignale der Analog/Digitalwandler (407) zur Erzeugung der Ansteuerungssignale für die Schrittmotoren (410) aufweist.

9. Stativ nach einem der Ansprüche 1 bis 8, **dadurch gekennzeichnet, daß** die Antriebe Getriebe aufweisen.

10. Stativ nach einem der Ansprüche 1 bis 9, **dadurch gekennzeichnet, daß** die möglichen Bewegungen um die einzelnen Achsen begrenzt sind.

11. Stativ nach einem der Ansprüche 3 bis 10, **dadurch gekennzeichnet, daß** die Sensoren (S1, S2) optische Aufnehmer mit kleinen Wegen aufweisen.

12. Stativ nach einem der Ansprüche 3 bis 10, **dadurch gekennzeichnet, daß** die Sensoren (S1, S2) kapazitive Aufnehmer aufweisen.

13. Stativ nach einem der Ansprüche 3 bis 10, **dadurch gekennzeichnet, daß** die Sensoren (S1, S2) induktive Aufnehmer mit kleinen Wegen aufweisen.

14. Stativ nach einem der Ansprüche 3 bis 10, **dadurch gekennzeichnet, daß** die Sensoren (S1, S2) Piezoelemente aufweisen.

## Claims

1. Stand with a surgical microscope, the microscope (111) having altogether up to six degrees of freedom and being fixed on a microscope mounting (109, 110) by means of elements of the stand (102, 103, 106, 108), which has two vertical pivoting axes (A1, A2), which are not subjected to any gravitational influence, and for the height adjustment a parallelogram linkage (105) with weight compensation, which are formed in such a way that they can move smoothly and have arresting brakes, the microscope being mounted on the stand by means of a third vertical axis (A3), which is not subjected to any gravitational influence, a first further axis (A4) perpendicular to said third vertical axis and a second further axis (A5) perpendicular to the first further axis, the further axes (A4, A5) being designed such that they are essentially perpendicular to the optical axis of the microscope objective (116) and can be adjusted by motor, and the further axis (A5) that is closer to the microscope permitting a lateral pivoting of the viewing direction (X direction) and the further axis (A4) that is arranged between the third vertical axis (A3) and the further axis (A5) that is closer to the microscope permitting a pivoting of the viewing direction forwards/backwards or upwards/downwards (Y direction).

2. Stand according to Claim 1, **characterized in that** the motor drives (410) are designed as servo drives.

3. Stand according to Claim 2, **characterized in that**, for controlling the servo drives, force or torque sensors (S1, S2) are arranged between the microscope (111) and at least one operating element (115) and generate signals corresponding to the size and direction of the force exerted on the operating elements, or the torque exerted, for the appropriate control of the motors (410).

4. Stand according to Claim 3, **characterized in that** the operating elements (115) are adjustable, without changing the position of the sensors (S1, S2) in relation to the microscope (111).

5. Stand according to Claim 3 or 4, **characterized in that** the sensors (S1, S2) are arranged in pairs in such a way that, when one sensor (S1, S2) is relieved by a force exerted on the operating element (115) by an operator, the other sensor (S2, S1) is loaded.

6. Stand according to Claim 5, **characterized in that** the sensors (S1, S2) are arranged in a bridge circuit (405) which emits a voltage determining the direction and speed of the motorized adjustment.

7. Stand according to Claim 6, **characterized in that** it has circuits (406-409) for converting the voltage into a supply voltage for the servo drive (204, 206).

8. Stand according to Claim 6, **characterized in that** it has stepping motors (410) for the servo drive (204, 206), analog/digital converters (407) for the voltage and signal circuits (408, 409) for the output signals of the analog/digital converters (407) for generating the activation signals for the stepping motors (410).

9. Stand according to one of Claims 1 to 8, **characterized in that** the drives have gear mechanisms.

10. Stand according to one of Claims 1 to 9, **characterized in that** the possible movements about the individual axes are limited.

11. Stand according to one of Claims 3 to 10, **characterized in that** the sensors (S1, S2) have optical pickups with small, displacements.

12. Stand according to one of Claims 3 to 10, **characterized in that** the sensors (S1, S2) have capacitive pickups.

13. Stand according to one of Claims 3 to 10, **characterized in that** the sensors (S1, S2) have inductive pickups with small displacements.

14. Stand according to one of Claims 3 to 10, **characterized in that** the sensors (S1, S2) have piezo elements.

## Revendications

1. Support avec un microscope chirurgical, le microscope (111) comportant en tout jusqu'à six degrés de liberté et étant fixé par l'intermédiaire d'éléments du support (102, 103, 106, 108) à une suspension (109, 110) qui comporte deux axes de pivotement (A1, A2) verticaux, qui ne sont pas influencés par la force de gravité, et un parallélogramme articulé (105) avec équilibrage de poids pour le réglage en hauteur, lesquels sont réalisés avec une mobilité aisée et avec des freins pour l'immobilisation, le microscope étant monté sur le support par l'intermédiaire d'un troisième axe (A3) vertical, qui n'est pas influencé par la force de gravité, d'un premier axe supplémentaire (A4) perpendiculaire à celui-ci et d'un deuxième axe supplémentaire (A5) perpendiculaire au premier axe supplémentaire, les axes supplémentaires (A4, A5) étant sensiblement perpendiculaires à l'axe optique de l'objectif (116) du microscope et étant conçus de manière réglable par moteur, et l'axe supplémentaire (A5) le plus proche du microscope (111) permettant un pivotement latéral du sens d'observation (direction X) et l'axe supplémentaire (A4), agencé entre le troisième axe (A3) vertical et l'axe supplémentaire (A5) le plus proche du microscope (111), permettant un pivotement du sens d'observation vers l'avant et l'arrière ou vers le haut et le bas (direction Y).

2. Support selon la revendication 1, **caractérisé en ce que** les entraînements motorisés (410) sont conçus sous forme de servomoteurs.

3. Support selon la revendication 2, **caractérisé en ce que**, pour la commande des servomoteurs, des capteurs de force ou de couple de rotation (S1, S2) sont agencés entre le microscope (111) et au moins un élément de commande (115), lesquels capteurs, en fonction de la valeur et de la direction de la force ou du couple de rotation exercés sur les éléments de commande, génèrent des signaux pour la commande appropriée des moteurs (410).

4. support selon la revendication 3, **caractérisé en ce que** les éléments de commande (115) sont réglables sans entraîner une variation de la position des capteurs (S1, S2) par rapport au microscope (111).

5. Support selon la revendication 3 ou 4, **caractérisé en ce que** les capteurs (S1, S2) sont agencés par paires, **en ce que**, sous l'effet d'une force exercée par une personne de service sur l'élément de commande (115), un capteur (S1, S2) est déchargé, l'autre (S2, S1) est sollicité.

6. Support selon la revendication 5, **caractérisé en ce que** les capteurs (S1, S2) sont agencés dans un circuit en pont (405), qui délivre une tension déterminant la direction et la vitesse du réglage motorisé.

7. Support selon la revendication 6, **caractérisé en ce qu'**il comporte des circuits (406-409) destinés à transformer la tension en une tension d'alimentation pour la servocommande (204, 206).

8. Support selon la revendication 6, **caractérisé en ce qu'**il comporte des moteurs pas à pas (410) pour la servocommande (204, 206), des convertisseurs analogique-numérique (407) pour la tension et des circuits de régénération des signaux (408, 409) pour les signaux de sortie des convertisseurs analogique-numérique (407) en vue de générer les signaux d'activation pour les moteurs pas à pas (410).

9. Support selon l'une quelconque des revendications 1 à 8, **caractérisé en ce que** les entraînements comportent des engrenages.

10. Support selon l'une quelconque des revendications 1 à 9, **caractérisé en ce que** les mouvements possibles autour des différents axes sont limités.

11. Support selon l'une quelconque des revendications 3 à 10, **caractérisé en ce que** les capteurs (S1, S2) comportent des capteurs optiques à courtes trajectoires.

12. Support selon l'une quelconque des revendications 3 à 10, **caractérisé en ce que** les capteurs (S1, S2) comportent des capteurs capacitifs.

13. Support selon l'une quelconque des revendications 3 à 10, **caractérisé en ce que** les capteurs (S1, S2) comportent des capteurs inductifs à courtes trajectoires.

14. Support selon l'une quelconque des revendications 3 à 10, **caractérisé en ce que** les capteurs (S1, S2) comportent des éléments piézo-électriques.
